# EUROPEAN PATENT APPLICATION

(11) **EP 2 818 095 A1**
(43) Date of publication of application: **31.12.2014**
(21) Application number: 14173669.4
(22) Date of filing: 24.06.2014
(51) Int. Cl.: A61B 1/00, A61B 1/04, A61B 1/24, G06T 5/00, G06T 5/50

(54) **Method for operating a dental camera**

(30) Priority: 24.06.2013 DE 102013106556
(71) Applicant: Qioptiq Photonics GmbH & Co. KG, 37081 Göttingen (DE)
(72) Inventor: Rauscher, Helmut, 83627 Warngau (DE)
(74) Representative: Wablat Lange Karthaus

(57) **Abstract**

In a method for operating of a dental camera to which having an illumination device with at least one light source for illuminating a tooth under examination with illuminating light, and also an image processing device are allocated, at least one first image and one second image are recorded, wherein between the first and the second image at least one property feature of the illuminating light is varied, preferably its angle of incidence onto the tooth, and wherein the images are compared by the image processing device in order to determine any overexposure.

## Description

### FIELD OF THE INVENTION

The invention relates to a method for operating of a dental camera.

### BACKGROUND OF THE INVENTION

Dental cameras are generally known and are employed largely in the dental field for purposes of examination and documentation.

To illuminate a tooth under examination with illuminating light, the dental camera cooperates with a lighting device with at least one light source. For processing of still pictures or moving pictures recorded by the dental camera, an image processing device is provided.

### SUMMARY OF THE INVENTION

Embodiments of the present invention provide a method of operating a dental camera. Briefly described, the present invention is directed to a method for operating a dental camera having an illumination device with at least one light source for illuminating a tooth under examination with illuminating light, as well as image processing device. The method includes the steps of recording at least one first image and one second image, varying at least one property feature of the illuminating light between the first and the second image, and determining an over exposure by comparing the images by the image processing device.

Various embodiments of the method for operating a dental camera are described in dependent claims 2-8 appended below.

Other systems, methods and features of the present invention will be or become apparent to one having ordinary skill in the art upon examining the following drawings and detailed description. It is intended that all such additional systems, methods, and features be included in this description, be within the scope of the present invention and protected by the accompanying claims.

### BRIEF DESCRIPTION OF THE DRAWINGS

The invention is explained in detail below based on the attached drawings which serve to illustrate one exemplary embodiment of the method according to the invention. All described features illustrated in the drawings and claimed in the patent claims each individually form the subject matter of the invention, either individually or in any suitable combination with each other, regardless of their combination in the patent claims and their back-references and even independently of their description and depiction in the drawings.
Figure 1 is a highly schematic and block diagram of one exemplary embodiment of a device to implement one exemplary embodiment of the method according to the invention.
Figure 2 shows two schematic images taken within the scope of the invented method, shown as examples, and one resulting, reflection-reduced image.
Figure 3 shows a sequence of images of reflection-reduced images taken within the scope of the invented method.

### DETAILED DESCRIPTION

Reference will now be made in detail to embodiments of the present invention, examples of which are illustrated in the accompanying drawings. Wherever possible, the same reference numbers are used in the drawings and the description to refer to the same or like parts

The invention is based on the problem of improving the image quality in the use of dental cameras. The invention is based on the finding that in the imaging of teeth there is frequent overexposure of the image owning to the smooth surface of the teeth, and this adversely impacts the image quality and evaluation of the recorded images. This applies all the more because the natural moisture on teeth additionally promotes the tendency to overexposure. Secondly, within the meaning of a high-quality image it is desirable to work at a high illumination strength of the light source, which further promotes the tendency to overexposure.

Taking these factors into account, the invention is based on the idea of improving the image quality of a dental camera by reducing or eliminating reflections in the recorded images.

For this purpose, the invention provides that at least one first image and one second image are recorded, wherein between the first and the second image at least one property feature of the illuminating light is varied, and wherein the images are compared by the image processing device in order to determine any overexposure.

For example and in particular, between the first image and the second image the angle of incidence of the illuminating light on the tooth under examination is the varied property feature of the illuminating light. The consequence is that an overexposure in the first image caused by the light source will appear at another location than in the second image. This can be detected in the image processing device and the image can be processed such that the overexposure is reduced or eliminated. For example, if the image processing device determines that an image region containing an overexposure in the first image is free of overexposure in the second image, then the overexposed image can be replaced by the image region of the second image, which is free of overexposure, for example, by replacing the overexposed pixels of the first image with non-overexposed pixels from the second image. In this manner overexposure can be reduced or eliminated, so that the image quality of the images recorded by means of the dental camera is significantly improved.

According to the invention it is also possible to vary other property features of the illuminating light between the first image and the second image. For example, according to the invention it is possible to vary the lighting intensity of the illuminating light between the first and the second images, so that in one image a stronger overexposure occurs than in the other image, in other words, in the one image a larger image region is overexposed than in the other image. In this manner it can be determined at least that an overexposure has occurred, so that for example in the image presented to a user of the dental camera, the overexposure can be marked so that it is evident to the user that the image information located at this site is only of limited predictive value with respect to the surface structure or 3D structure of the examined tooth.

One favorable embodiment of the invention provides that an image region in which an overexposure is found is subjected to image processing by the image processing device to reduce or eliminate the overexposure. Corresponding algorithms of image processing and pattern recognition are widely available and can be employed without change within the scope of the invention. Details on corresponding algorithms are generally known to the ordinary person skilled in the art and thus will not be discussed further at this point.

As already discussed, different property features of the illuminating light can be changed in accordance with the purpose of the invention. One particularly advantageous embodiment of the invention provides that between the first image and the second image the angle of incidence of the illuminating light on the tooth under examination is the varied property feature of the illuminating light. Given an appropriate selection of the angle of incidence, accordingly overexposure occurs at different locations of the image, so that in particular it will be possible when detecting an overexposure in an image which is not detected in the other image, an image region of the one image containing the overexposure can be replaced by a corresponding image region of the other image which does not have overexposure, as is called for in another embodiment of the invention. In this manner the image quality of the dental camera is significantly improved.

Depending on the particular requirements, the dental camera can record still images or moving images.

An additional favorable embodiment of the invention provides means which determine whether a relative motion between the dental camera and the tooth being imaged has occurred in the time period between the first and the second images. Changes in position of the tooth relative to the dental camera in this embodiment can be reflected in the image processing of an overexposed image, so that the image quality is further improved.

A refinement of the aforementioned embodiment provides that sensors means are the means which determine whether a relative motion between the dental camera and the tooth being imaged has occurred between the first and the second images. By means of suitable sensors, relative motions between tooth and dental camera can be determined according to their type and scope and then taken into account in the image processing.

In order to structure the sensor means as simply as possible in the aforementioned embodiment, one refinement of the aforementioned embodiment provides that the sensor means features at least one motion sensor allocated to the dental camera, said sensor transmitting signals to the image processing device. A corresponding motion sensor, which can detect both the absolute position of the camera head of the dental camera as well as position changes, can be attached to the camera head of the dental camera, for example. Corresponding motion sensors are available as relatively simple and low-cost, standard components.

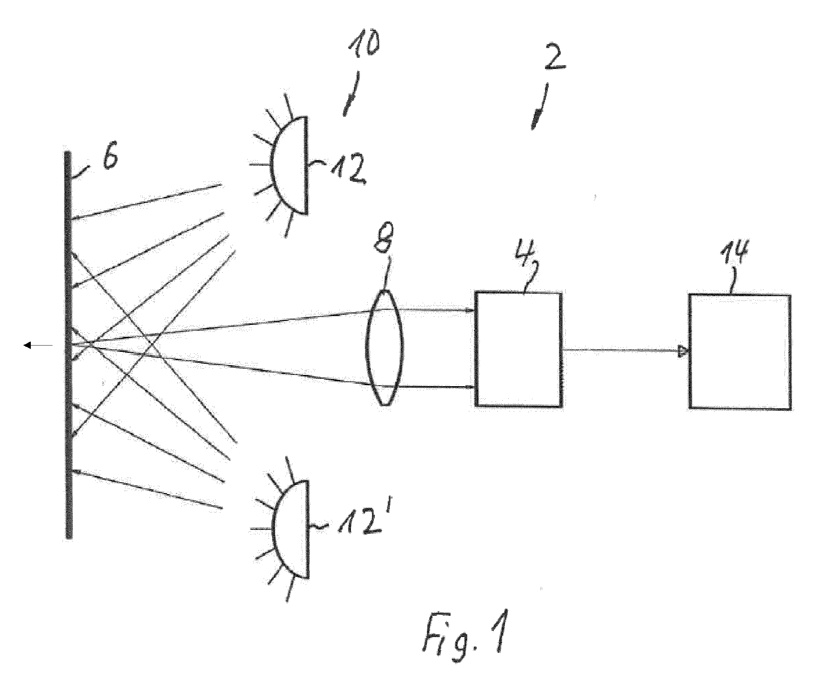

Figure 1 depicts one exemplary embodiment of a device 2 to implement one exemplary embodiment of the invented method for operating of a dental camera 4 which is used for imaging of a tooth which is symbolized in Figure 1 by a plane 6. For imaging of the tooth 6 an observation lens 8 is allocated to the dental camera 4. An illumination device 10 for illuminating of the tooth being imaged with illuminating light is allocated to the dental camera 4, wherein the illuminating device 10 in the illustrated, exemplary embodiment has two light sources 12, 12', which in the illustrated embodiment are arranged spatially apart from each other and emit light at different angles of incidence on the tooth 6.

Furthermore, an image processing device 14 is allocated to the dental camera 4 and is used for processing of the images captured by means of the dental camera 4. In the illustrated embodiment, the dental camera 4 is provided for recording of still images. However, the dental camera 4 can also be provided according to the particular circumstances and within the scope of the invention method, for capturing of moving images.

The method according to the invention is implemented by means of the device 2 according to the invention as follows:
Within the scope of the invented method, initially a first image is captured of the tooth 6, wherein the light source 12 is switched on and the light source 12' is switched off.

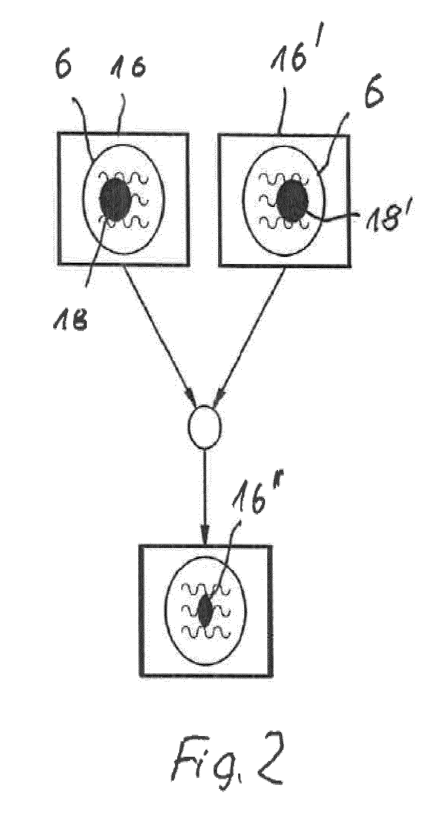

Figure 2 shows a corresponding image 16 under the assumption that an overexposure occurs in image 16, which is indicated in Figure 2 by reference number 18. The tooth 6 is symbolized in Figure 2 as a circular shape.

After capturing of the first image 16, the light source 12 is switched off and light source 12' is switched on, so that as is evident in Figure 1, the angle of incidence of the illuminating light on the tooth 6 is changed according to the invention. In this illumination constellation a second image 16' is captured, wherein the location of the overexposure 18' has changed according to the changed angle of incidence of the illuminating light. The images 16, 16' captured in this manner are supplied to the image processing device 14.

Based on the data representing images 16, 16' the image processing device 14 can determine if and at what location any overexposure 18, 18' has occurred in the images 16, 16'. An overexposure can be detected, for example, in that the lights are "burnt" in the affected image region, in other words, a corresponding number of pixels is located at the far right edge of the histogram.

According to the invention, the image region in which an overexposure is detected is subjected in the image processing device 14 to image processing for removal or reduction of the overexposure.

In the exemplary embodiment depicted in Figure 2, a circumstance occurs in which the overexposures only partly overlap. In the region of the overlap, both images are overexposed so that in this region a reconstruction of the first image 16 with corresponding pixels of the second image 16' is not possible, and accordingly a residual overexposure 16" is present in an image produced from a combination of images 16, 16'. In those regions where an overexposure is present only in one of the images 16, 16', the overexposed image region of the one image can be replaced by the non-overexposed image region of the other image, as illustrated in Figure 2 on image 16".

The resulting image 16" is thus much lower in reflection compared to images 16, 16', so that the image quality is significantly improved.

By means of the corresponding changes to the angle of incidence of the illuminating light, according to the invention overexposures can be largely reduced or even eliminated.

Purely as an example in Figure 2 two light sources 12, 12' are depicted. However, according to the invention it is also possible to use more than two light sources, for example, in the form of an LED-line or an LED-crown, wherein the individual LEDs are switched on in sequence and each one can capture an image of the tooth. In this manner, according to the invention more than two images can be used to produce a low-reflective image, corresponding to the number of available light sources emitting light at different angles of incidence.

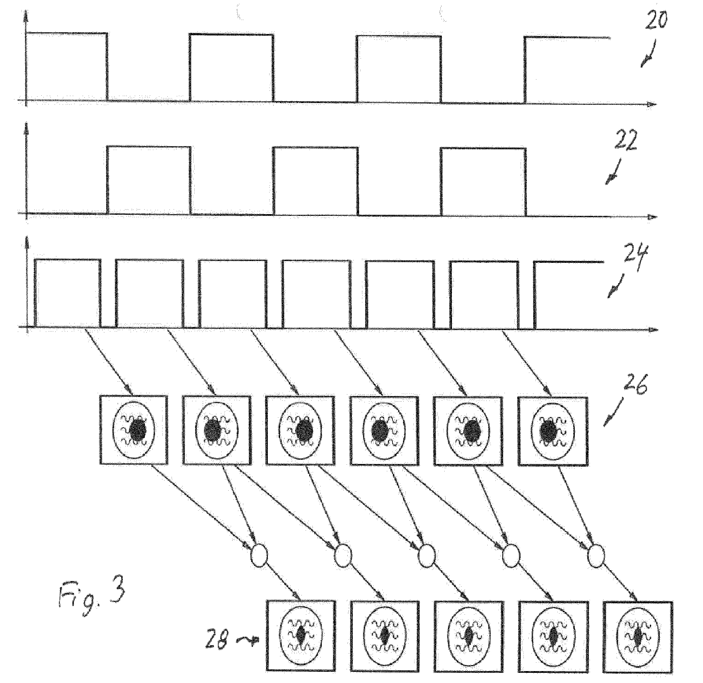

Figure 3 illustrates a sequence of image generation with reflection reduction, wherein the sequence of illumination by the light source 12 is denoted by reference number 20, and the time progression of illumination by light source 12' is denoted by reference number 22. It is evident that the light sources 12, 12' are switched on alternately. Reference number 24 in Figure 3 denotes the opening and closing of the aperture of the digital camera 4, wherein it is evident that an image is captured each time after the switch between light sources 12, 12'.

Reference number 26 denotes a sequence of images captured by means of the dental camera 4, whereas reference number 28 denotes a sequence of reduced-reflection images captured according to the inventive method.

The method according to the invention allows an improvement in the image quality of a dental camera in a simple and reliable manner.

It will be apparent to those skilled in the art that various modifications and variations can be made to the structure of the present invention without departing from the scope or spirit of the invention. In view of the foregoing, it is intended that the present invention cover modifications and variations of this invention provided they fall within the scope of the following claims and their equivalents.

## Claims

1. A method for operating a dental camera to which an illumination device with at least one light source for illuminating a tooth under examination with illuminating light, as well as with an image processing device are allocated, wherein at least one first image and one second image are recorded, wherein between the first and the second image at least one property feature of the illuminating light is varied, and wherein the images are compared by the image processing device in order to determine any overexposure.

2. The method according to Claim 1, **characterized in that** an image region in which an overexposure is determined is subjected to image processing by the image processing device to eliminate or to reduce the overexposure.

3. The method according to Claim 1 or 2, **characterized in that** between the first image and the second image the angle of incidence of the illuminating light on the tooth under examination is the varied property feature of the illuminating light.

4. The method according to any of the preceding claims, **characterized in that** upon determination of an overexposure in one image, which is not detected, at least in regions, in the other image, an image region containing the overexposure of the one image is replaced by a corresponding image region of the other image.

5. The method according to any of the preceding claims, **characterized in that** the dental camera records still images or moving images.

6. The method according to any of the preceding claims, **characterized in that** means are provided which determine whether a relative motion between the dental camera and the tooth being examined has occurred in the time period between the first and the second image.

7. The method according to Claim 6, **characterized in that** the means which determine whether a relative motion between the dental camera and the tooth being examined has occurred between the first and the second image include sensor means.

8. The method according to Claim 7, **characterized in that** the sensor means features at least one motion sensor allocated to the dental camera, said sensor being in signal transmission connection with the image processing device.
